# EUROPEAN PATENT APPLICATION

(11) **EP 4 691 550 A1**
(43) Date of publication of application: **11.02.2026**
(21) Application number: 24793095.1
(22) Date of filing: 16.02.2024
(51) Int. Cl.: A61N 1/40, A61N 1/08, A61N 1/06, A61N 1/32, A61F 7/00, A61B 18/00

(54) **METHOD OF OPERATION OF TIP AND VALVE FOR SKIN CARE, AND HANDPIECE COMPRISING SAME**

(30) Priority: 18.04.2023 KR 20230050724
(71) Applicant: Jeisys Medical Inc., Seoul 08501 (KR)
(72) Inventor: PARK, Jongseung, Seoul 08501 (KR); AN, Kyoungho, Seoul 08501 (KR)
(74) Representative: dompatent
(86) International application number: PCT/KR2024/095271
(87) International publication number: WO 2024/219911

(57) **Abstract**

The present disclosure may include a housing; an injection part provided in the housing and including a refrigerant delivery path formed to inject a cooling gas; a discharge part coupled to the injection part and including a discharge path formed to discharge the cooling gas to outside; and a valve coupled to the injection part and the discharge part and configured to block external air flowing in through the refrigerant delivery path and discharge the cooling gas to the outside through the discharge path while retaining the cooling gas, and configured to open and close according to a pressure of the cooling gas.

## Description

### [TECHNICAL FIELD]

The present disclosure relates to a skin care tip, a method for operating a valve, and a hand piece including the same.

### [BACKGROUND ART]

A high-frequency treatment device is a treatment device related to skin beauty that generates heat by high-frequency output to the skin tissue of the treatment target, which causes collagen fiber degeneration and contraction, thereby producing collagen regeneration and wrinkle improvement effects.

Since the high-frequency treatment device operates by generating high frequency, heat may be generated in the high-frequency electrode when outputting high frequency.

In addition, when the high-frequency treatment device performs cooling of the high-frequency electrode using a refrigerant during the treatment process, sufficient cooling of the high-frequency electrode needs to be achieved, so the refrigerant needs to be used continuously.

Therefore, the conventional high-frequency treatment device needs use the refrigerant continuously, and since the refrigerant vaporizes quickly, the tip temperature rises quickly, which increases the gas consumption, so the cost of using the refrigerant increases.

In addition, the conventional high-frequency treatment device cannot maintain a low internal temperature, so the cooling efficiency is low.

### [DETAILED DESCRIPTION OF THE INVENTION]

### [TECHINICAL PROBLEM]

The present disclosure is to prevent pain and burns caused by heat generation in a high-frequency electrode in advance.

In addition, the present disclosure is to prevent increases in refrigerant consumption and usage costs in advance.

In addition, the present disclosure is to prevent freezing inside in advance.

In addition, the present disclosure is to maintain a low internal temperature to improve cooling efficiency.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a skin care tip may include a housing; an injection part provided in the housing and including a refrigerant delivery path formed to inject a cooling gas; a discharge part coupled to the injection part and including a discharge path formed to discharge the cooling gas to outside; and a valve coupled to the injection part and the discharge part and configured to block external air flowing in through the refrigerant delivery path and discharge the cooling gas to the outside through the discharge path while retaining the cooling gas, and configured to open and close according to a pressure of the cooling gas.

Furthermore, the skin care tip may further include an injection control module mounted on the injection part and controlling an amount of the cooling gas injected according to the pressure of the cooling gas.

Furthermore, the valve may maintain a closing operation until the pressure of the cooling gas reaches a preset reference pressure, and open when the reference pressure is reached.

Furthermore, the valve may open at an opening level according to a preset pressure range when the pressure of the cooling gas reaches the reference pressure.

Furthermore, based on the pressure of the cooling gas being in a preset first pressure range, the valve may open at a preset micro-opening level in conjunction with the first pressure range.

Furthermore, based on the pressure of the cooling gas being in a preset second pressure range, the valve may open at a preset half-opening level in conjunction with the second pressure range.

Furthermore, based on the pressure of the cooling gas being in a preset third pressure range, the valve may open at a preset full-opening level in conjunction with the third pressure range.

Furthermore, the valve may be at least one of a check valve or a solenoid valve.

Furthermore, a hand piece equipped with a skin care tip may be further provided.

Furthermore, in another aspect of the present disclosure, a method of operating a valve included in a skin care tip may include detecting a pressure of a cooling gas injected through a refrigerant delivery path of the skin care tip; maintaining a closing operation when a pressure of the cooling gas does not reach a preset reference pressure; and opening at an opening level according to a preset pressure range when the pressure of the cooling gas reaches the preset reference pressure.

Furthermore, a computer program stored in a computer-readable recording medium for executing the present disclosure may be further provided.

Furthermore, a computer-readable recording medium recording a computer program for executing a method for executing the present disclosure may be further provided.

### [ADVANTAGEOUS EFFECTS OF THE INVENTION]

The present disclosure provides the effect of preventing pain and burns caused by heat generation in the high-frequency electrode.

Furthermore, the present disclosure provides the effect of preventing increases in refrigerant consumption and usage costs.

Furthermore, the present disclosure provides the effect of preventing internal freezing.

Furthermore, the present disclosure provides the effect of maintaining a low internal temperature, thereby improving cooling efficiency.

The effects of the present disclosure are not limited to the effects mentioned above, and other effects not mentioned will be clearly understood by those skilled in the art from the description.

### [DESCRIPTION OF THE DRAWINGS]

FIGS. 1 to 4 are diagrams illustrating an example of a configuration of a skin care tip according to the present disclosure.
FIGS. 5 to 8 are diagrams illustrating another example of a configuration of a skin care tip according to the present disclosure.
FIGS. 9 to 12 are diagrams illustrating still another example of a configuration of a skin care tip according to the present disclosure.
FIG. 13 illustrates a configuration of a control module of a hand piece equipped with a skin care tip according to the present disclosure.
FIGS. 14 and 15 are flowcharts illustrating an example of an operation method of a valve included in a skin care tip according to the present disclosure.
FIG. 16 is a flowchart illustrating another example of an operation method of a valve included in a skin care tip according to the present disclosure.

### [BEST MODE]

In the drawings, the same reference numeral refers to the same element. This disclosure does not describe all elements of embodiments, and general contents in the technical field to which the present disclosure belongs or repeated contents of the embodiments will be omitted. The terms, such as "module, module, member, and block" may be embodied as hardware or software, and a plurality of "modules, modules, members, and blocks" may be implemented as one element, or a module, a module, a member, or a block may include a plurality of elements.

Throughout this specification, when a part is referred to as being "connected" to another part, this includes "direct connection" and "indirect connection", and the indirect connection may include connection via a wireless communication network. Furthermore, when a certain part "includes" a certain element, other elements are not excluded unless explicitly described otherwise, and other elements may in fact be included.

Furthermore, when a certain part "includes" a certain element, other elements are not excluded unless explicitly described otherwise, and other elements may in fact be included.

In the entire specification of the present disclosure, when any member is located "on" another member, this includes a case in which still another member is present between both members as well as a case in which one member is in contact with another member.

The terms "first," "second," and the like are just to distinguish an element from any other element, and elements are not limited by the terms.

The singular form of the elements may be understood into the plural form unless otherwise specifically stated in the context.

Identification codes in each operation are used not for describing the order of the operations but for convenience of description, and the operations may be implemented differently from the order described unless there is a specific order explicitly described in the context.

Hereinafter, operation principles and embodiments of the present disclosure will be described with reference to the accompanying drawings.

The control module according to the present disclosure in this specification includes various devices that may perform computational processing and provide results to a user. For example, the control module according to the present disclosure may include a computer, a server device, and a portable terminal, or may be in the form of one of them.

Here, the computer may include, for example, a notebook, a desktop, a laptop, a tablet PC, a slate PC, and the like mounted with a web browser.

The server device is a server that communicates with an external device to process information, and may include an application server, a computing server, a database server, a file server, a mail server, a proxy server, and a web server.

A portable terminal is a wireless communication device that ensures portability and mobility, and may include all kinds of handheld-based wireless communication devices such as PCS (Personal Communication System), GSM (Global System for Mobile communications), PDC (Personal Digital Cellular), PHS (Personal Handyphone System), PDA (Personal Digital Assistant), IMT (International Mobile Telecommunication)-2000, CDMA (Code Division Multiple Access)-2000, W-CDMA (W-Code Division Multiple Access), WiBro (Wireless Broadband Internet) terminal, a smart phone, and the like, and a wearable device such as at least one of a watch, a ring, bracelets, anklets, a necklace, glasses, contact lenses, or a head-mounted device (HMD).

A skin care tip according to the present disclosure may include an injection part provided in a housing and including a refrigerant delivery path formed to inject a cooling gas; a discharge part coupled to the injection part and including a discharge path formed to discharge the cooling gas to outside; and a valve coupled to the injection part and the discharge part and configured to block external air flowing in through the refrigerant delivery path and discharge the cooling gas to the outside through the discharge path while retaining the cooling gas, and configured to open and close according to a pressure of the cooling gas.

The skin care tip may prevent pain and burns caused by heat generation in a high-frequency electrode in advance, prevent an increase in refrigerant consumption and usage cost in advance, prevent freezing inside in advance, and improve cooling efficiency by maintaining a low internal temperature.

Hereinafter, the skin care tip will be described in detail.

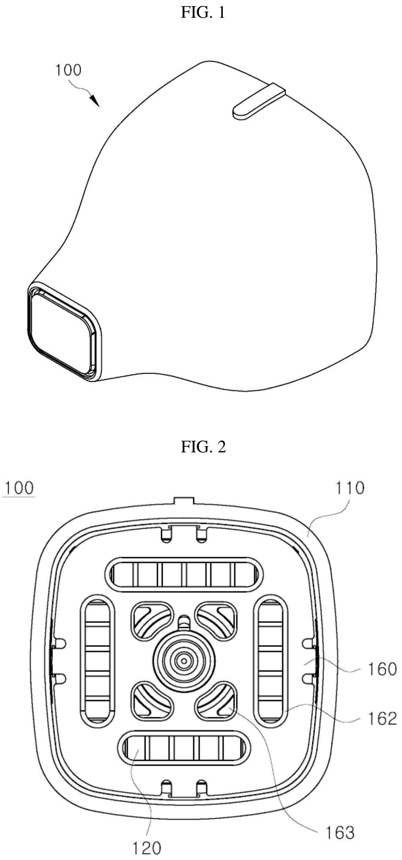

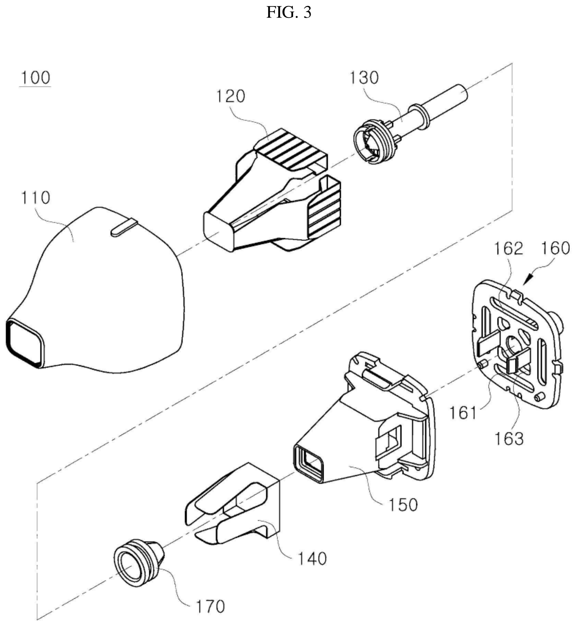

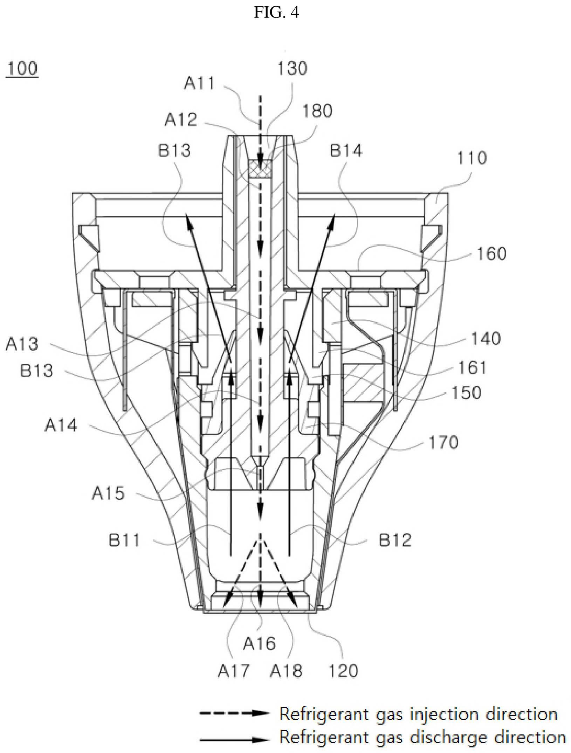

FIGS. 1 to 4 are diagrams illustrating an example of a configuration of a skin care tip according to the present disclosure.

Referring to FIGS. 1 to 4, a skin care tip 100 may include a first housing 110, a first injection part 130, a first discharge part 140, 150, and 160, and a first valve 170.

The first housing 110 may discharge a cooling gas and block an inside and an outside while preventing heat exchange between the inside and the outside. The first injection part 130 may be provided in the first housing 110 and may include a refrigerant delivery path A11 to A18 formed to inject a cooling gas. For example, the first injection part 130 may be provided as an ejector, but is not limited thereto, and may be provided as any member capable of injecting a cooling gas that is high pressure and has a low boiling point and is in a liquid state. In addition, the first injection part 130 may be provided inside the housing 110.

The first discharge part 140, 150, and 160 may be coupled with the first injection part 130 and may include a discharge path B11 to B14 formed to discharge the cooling gas to the outside. Here, the first discharge part 140, 150, and 160 may include a first guide member 140, a first cover 150, and a first fastening member 160. In this case, the skin care tip 100 may be provided so that the first injection part 130, the first guide member 140, and the first cover 150 are inserted into the first housing 110, and may be coupled with the first housing 110 by the first fastening member 160.

Here, the first guide member 140 may be provided to guide the first injection part 130 and the first valve 170 and be coupled to the housing 110. The first cover 150 may be provided to protect the first injection part 130, the first valve 170, and the first guide member 140. The first fastening member 160 may be provided to be fastened to the first cover 150.

For example, the first fastening member 160 may include at least one first fastening protrusion 161 to fasten the first cover 150. In addition, the first fastening member 160 may further include at least one first connecting hole 162 to electrically connect an external device, such as a hand piece, to a portion of the first electrode member 120. In this case, the first electrode member 120 may be a member that applies radio frequency (RF) to the dermis layer of the skin. For example, the first electrode member 120 may be a flat electrode for transmitting RF power to a flexible film-type PCB with a very thin thickness, and may serve to transmit low temperature generated by vaporization of the refrigerant to the opposite side of the film (skin) to perform heat exchange (skin cooling). In addition, the first fastening member 160 may further include at least one first discharge hole 163 so that the cooling gas is discharged to the outside by the opening operation of the first valve 170.

The first valve 170 may be coupled with the first injection part 130 and the first discharge part 140, 150, and 160, and may open and close depending on the pressure of the cooling gas to block external air flowing in through the refrigerant delivery path A11 to A18, and discharge the cooling gas to the outside through the discharge path B11 to B14 while retaining the cooling gas. For example, the first valve 170 may include a check valve and an electronic valve, and the electronic valve includes a solenoid valve, but is not limited thereto. Additionally, the solenoid valve may be provided in the hand piece, and may discharge the refrigerant when the temperature detected by the temperature sensor provided in the tip reaches a preset target temperature, thereby cooling the temperature down to below the preset target temperature.

The skin care tip 100 according to the present disclosure may deliver cooling gas through the refrigerant delivery path A11 to A18 based on an internal space formed by the first housing 110, the first electrode member 120, the first injection part 130, the first discharge part 140, 150, and 160, and the first valve 170, and may discharge the cooling gas to the outside through an opening operation of the first valve 170 and the discharge path B11 to B14. Here, the discharge path B11 to B14 is a path for discharging the gas used for cooling to the outside, and may serve to prevent external air containing moisture from flowing into the interior of the first injection part 130 having a low temperature. In this case, the vaporized gas is discharged to the outside through the first valve 170 along the discharge path B11 to B14, and the gas passing through the first valve 170 may not be re-introduced into the inside of the first injection part 130. In addition, the first valve 170 may prevent the inflow of warm outside air, prevent freezing inside, and maintain the inside at a lower temperature.

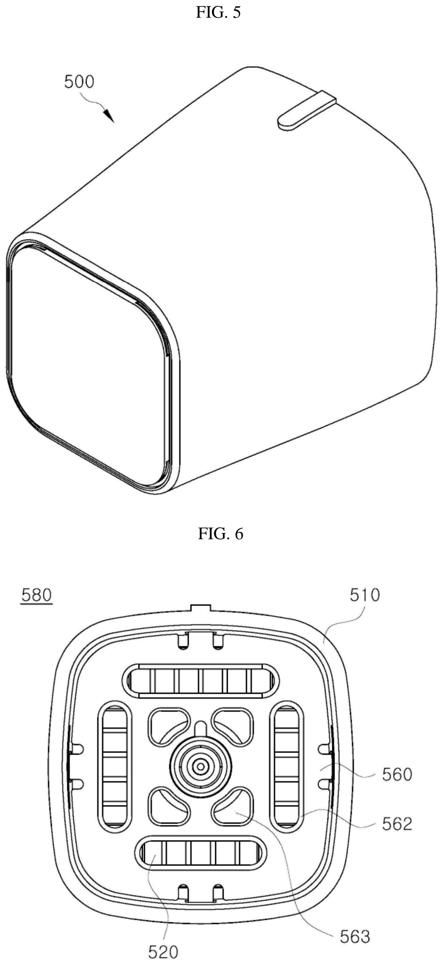

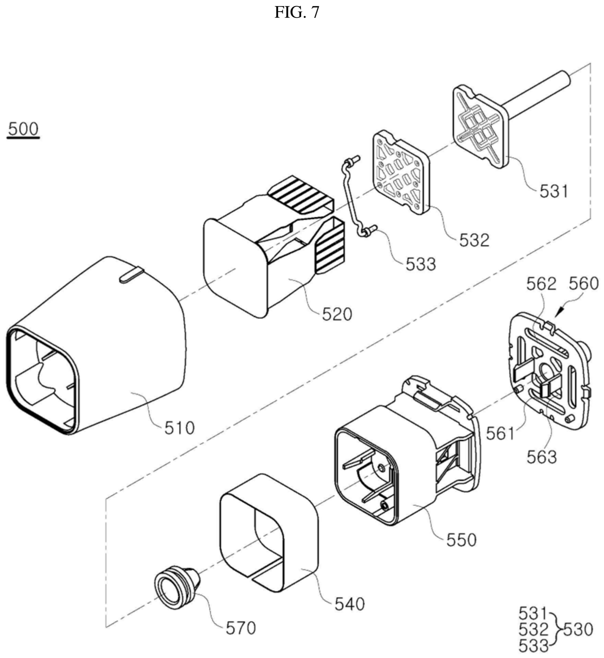

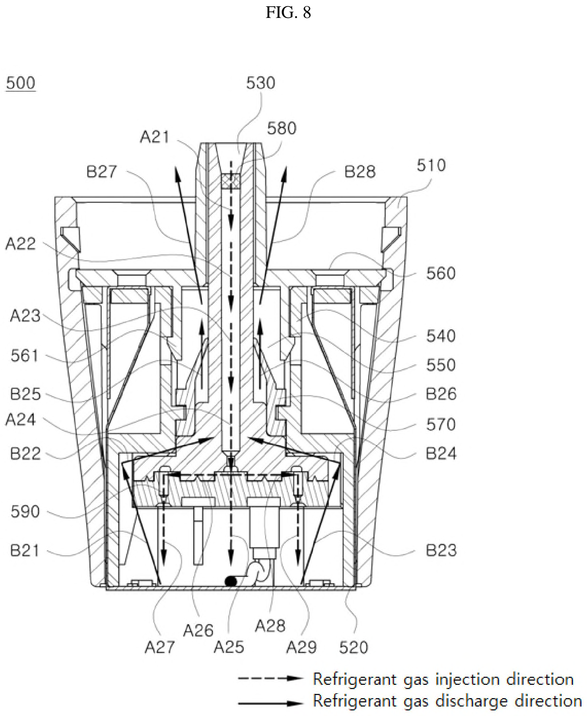

FIGS. 5 to 8 are diagrams illustrating another example of a configuration of a skin care tip according to the present disclosure.

Referring to FIGS. 5 to 8, a skin care tip 500 may include a second housing 510, a second injection part 530, a second discharge part 540, 550, and 560, and a second valve 570.

The second housing 510 may discharge cooling gas and block the inside and the outside while preventing heat exchange between the inside and the outside. The second injection part 530 may be provided in the second housing 510 and may include a refrigerant delivery path A21 to A29 formed to inject cooling gas. For example, the second injection part 530 may be provided as an ejector. Here, the second injection part 530 may include an ejector body 531, a diffusion plate 532, and a fixing member 533. In this case, the ejector body 531 may be fixed to the diffusion plate 532 by the fixing member 533. The second injection part 530 may evenly diffuse and inject the cooling gas output from the ejector body 531 through the diffusion plate 532 including a plurality of holes h1 at a regular interval in a uniform amount. Meanwhile, the second injection part 530 is not limited thereto, and may be provided with any member capable of evenly diffuse and injecting the cooling gas in a liquid state with a low boiling point and high pressure in a uniform amount. In addition, the second injection part 530 may be provided inside the housing 510.

In addition, a second injection nozzle 590 may be further provided in the second injection part 530. For example, the second injection nozzle 590 may be a fine nozzle arranged at a regular interval. In this case, the second injection nozzle 590 may have a wide injection angle to uniformly cool the entire area of the second electrode member 520 according to the area of the second electrode member 520. As the cooling gas is sprayed like a large-area spray by the second injection nozzle 590, the entire area of the second electrode member 520 may be uniformly cooled, and the cooling gas sprayed by the second injection nozzle 590 attaches to the second electrode member 520 and vaporizes, thereby lowering the internal temperature.

The second discharge part 540, 550, and 560 may be combined with the second injection part 530 and may include a discharge path B21 to B28 formed to discharge the cooling gas to the outside. Here, the second discharge part 540, 550, and 560 may include a second guide member 540, a second cover 550, and a second fastening member 560. In this case, the skin care tip 500 may be provided so that the second injection part 530, the second guide member 540, and the second cover 550 are inserted into the second housing 510, and may be coupled to the second housing 510 by the second fastening member 560.

Here, the second guide member 540 may be provided so that the second injection part 530 and the second valve 570 are guided and coupled to the housing 510. The second cover 550 may be provided to protect the second injection part 530, the second valve 570, and the second guide member 540. The second fastening member 560 may be arranged to be fastened to the second cover 550.

For example, the second fastening member 560 may include at least one second fastening protrusion 561 to fasten the second cover 550. In addition, the second fastening member 560 may further include at least one second connection hole 562 to electrically connect an external device, such as a hand piece, to a portion of the second electrode member 520. In this case, the second electrode member 520 may be a member that applies radio frequency (RF) to the dermis layer of the skin. For example, the second electrode member 520 may be a flat electrode for transmitting RF power to a flexible film-type PCB with a very thin thickness, and may serve to transmit low temperature generated by vaporization of a refrigerant to the opposite side of the film (skin) to perform heat exchange (skin cooling). In addition, the second fastening member 560 may further include at least one second discharge hole 563 so that the cooling gas is discharged to the outside by the opening operation of the second valve 570.

The second valve 570 may be connected to the second injection part 530 and the second discharge part 540, 550, and 560, and may be opened and closed according to the pressure of the cooling gas to block the outside air flowing in through the refrigerant delivery path A21 to A29 while retaining the cooling gas, and discharge the cooling gas to the outside through the discharge path B21 to B28. For example, the second valve 570 may include a check valve and an electronic valve, and the electronic valve includes a solenoid valve, but is not limited thereto. Additionally, the solenoid valve may be provided in the hand piece, and may discharge the refrigerant when the temperature detected by the temperature sensor provided in the tip reaches the preset target temperature, thereby cooling the temperature down to below the preset target temperature.

The skin care tip 500 according to the present disclosure may evenly distribute and deliver a cooling gas in a uniform amount through the refrigerant delivery path A11 to A18 based on the internal space formed by the second housing 510, the second electrode member 520, the second injection part 530, the second discharge part 540, 550, and 560, and the second valve 570, and may discharge the cooling gas to the outside while retaining it through the opening operation of the second valve 570 and the discharge path B21 to B28. Here, the discharge path B21 to B28 is a path for discharging the gas used for cooling to the outside, and may serve to prevent external air containing moisture from flowing into the interior of the second injection part 530 having a low temperature. At this time, the vaporized gas is discharged to the outside through the second valve 570 along the discharge path B21 to B28, and the gas passing through the second valve 570 may not be re-introduced into the inside of the second injection part 530. In addition, the second valve 570 may prevent the inflow of warm outside air, prevent freezing inside, and maintain the inside at a lower temperature.

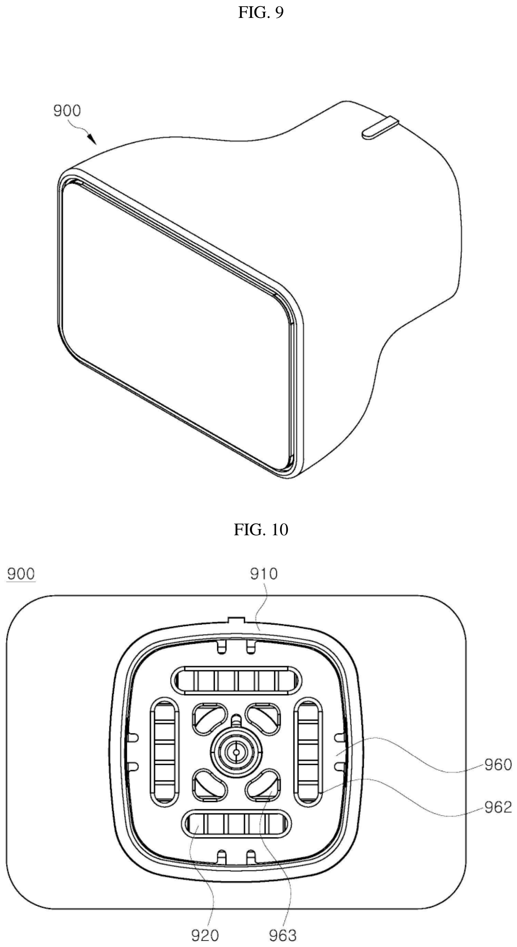

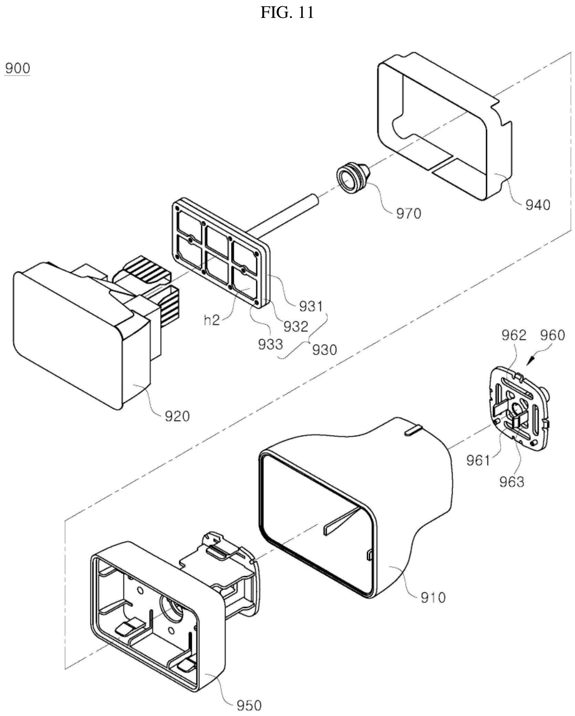

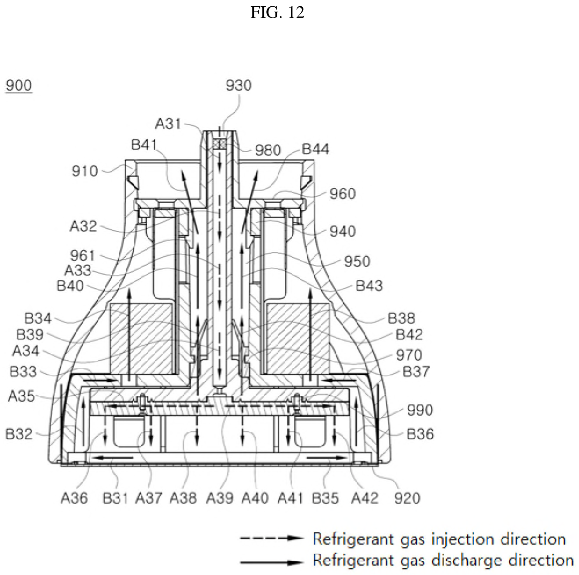

FIGS. 9 to 12 are diagrams illustrating still another example of a configuration of a skin care tip according to the present disclosure.

Referring to FIGS. 9 to 12, a skin care tip 900 may include a third housing 910, a third injection part 930, a third discharge part 940, 950, and 960, and a third valve 970.

The third housing 910 may discharge cooling gas and block the inside and the outside while preventing heat exchange between the inside and the outside. The third injection part 930 may be provided in the third housing 910 and may include a refrigerant delivery path A31 to A42 formed to inject cooling gas. For example, the second injection part 930 may be provided as an ejector. Here, the third injection part 930 may include an ejector body 931, a diffusion plate 932, and a fixing member 933. In this case, the ejector body 931 may be fixed to the diffusion plate 932 by the fixing member 933. This third injection part 930 may evenly and evenly inject the cooling gas output from the ejector body 931 by spreading it evenly and over a large area through the diffusion plate 932 including a plurality of holes h2 at regular intervals. Meanwhile, the third injection part 930 is not limited thereto, and may be provided with any member that may evenly and evenly inject the cooling gas in a liquid state while having a low boiling point and high pressure by spreading it evenly and over a large area. In addition, the third injection part 930 may be provided inside the housing 910.

In addition, a third injection nozzle 990 may be further provided in the third injection part 930. For example, the third injection nozzle 990 may be a fine nozzle arranged at a regular interval. At this time, the third injection nozzle 990 may have a wide injection angle to uniformly cool the entire area of the third electrode member 920 according to the area of the third electrode member 920. As the cooling gas is sprayed like a large-area spray by the third injection nozzle 990, the entire area of the third electrode member 920 may be uniformly cooled, and the cooling gas sprayed by the third injection nozzle 990 attaches to the third electrode member 920 and vaporizes, thereby lowering the internal temperature.

The third discharge part 940, 950, and 960 may be combined with the third injection part 930 and may include a discharge path B31 to B44 formed to discharge the cooling gas to the outside. Here, the third discharge part 940, 950, and 960 may include a third guide member 940, a third cover 950, and a third fastening member 960. In this case, the skin care tip 900 may be provided so that the third injection part 930, the third guide member 940, and the third cover 950 are inserted into the third housing 910, and may be coupled to the third housing 910 by the third fastening member 960.

Here, the third guide member 940 may be provided so that the third injection part 930 and the third valve 970 are guided and coupled to the third housing 910. The third cover 950 may be provided to protect the third injection part 930, the third valve 970, and the third guide member 940. The third fastening member 960 may be arranged to be fastened to the third housing 910 and the third cover 950.

For example, the third fastening member 960 may include at least one third fastening protrusion 961 to fasten the third cover 950 while contacting the third housing 910. In addition, the third fastening member 960 may further include at least one third connecting hole 962 to electrically connect an external device, such as a hand piece, to a portion of the third electrode member 920. At this time, the third electrode member 920 may be a member that applies radio frequency (RF) to the dermal layer of the skin. For example, the third electrode member 920 may be a flat electrode for transmitting RF power to a flexible film-type PCB with a very thin thickness, and may transfer the low temperature generated by the vaporization of the refrigerant to the opposite side of the film skin to perform heat exchange skin cooling. In addition, the third fastening member 960 may further include at least one third discharge hole 963 so that the cooling gas is discharged to the outside by the opening operation of the third valve 970.

The third valve 970 is coupled to the third injection part 930 and the third discharge part 940, 950, and 960 and may open and close depending on the pressure of the cooling gas to block the outside air flowing in through the refrigerant delivery path A31 to A42 and discharge the cooling gas to the outside through the discharge path B31 to B44 while retaining the cooling gas. For example, the third valve 970 may include a check valve and an electronic valve, and the electronic valve may include a solenoid valve, but is not limited thereto. Additionally, the solenoid valve may be provided in the handpiece, and may discharge a refrigerant when the temperature detected by the temperature sensor provided in the tip reaches a preset target temperature, thereby cooling the temperature down to below the preset target temperature.

The skin care tip 900 according to the present disclosure may evenly distribute and deliver cooling gas in a large amount over a uniform area through the refrigerant delivery path A31 to A42 based on the internal space formed by the third housing 910, the third electrode member 920, the third injection part 930, the third discharge part 940, 950, and 960, and the third valve 970, and may discharge the cooling gas to the outside while retaining it through the opening operation of the third valve 970 and the discharge path B31 to B44. Here, the discharge path B31 to B44 is a path for discharging the gas used for cooling to the outside, and may serve to prevent external air containing moisture from flowing into the interior of the third injection part 930 having a low temperature. At this time, the vaporized gas is discharged to the outside through the third valve 970 along the discharge path B31 to B44, and the gas passing through the third valve 970 may not be re-introduced into the inside of the third injection part 930. In addition, the third valve 970 may prevent the inflow of warm outside air, prevent freezing inside, and maintain the inside at a lower temperature.

At least one of the first to third valves 170, 570, or 970 of the skin care tip 100, 500, and 900 according to the present disclosure may maintain a closed operation until the pressure of the cooling gas reaches a preset reference pressure, and may also open when the reference pressure is reached. In this case, at least one of the first to third valves 170, 570, or 970 may open at an opening level according to a preset pressure range when the pressure of the cooling gas reaches the reference pressure.

For example, at least one of the first to third valves 170, 570, or 970 may open at a preset micro-opening level in conjunction with the first pressure range when the pressure of the cooling gas is in a preset first pressure range. In this case, the first pressure range may be a low level pressure range.

For another example, at least one of the first to third valves 170, 570, or 970 may open at a preset half-opening level in conjunction with the second pressure range when the pressure of the cooling gas is in a second pressure range higher than the preset first pressure range. At this time, the second pressure range may be a middle level pressure range.

For still another example, at least one of the first to third valves 170, 570, or 970 may be opened to a preset full-opening level in conjunction with the third pressure range when the pressure of the cooling gas is in a third pressure range higher than the preset second pressure range. At this time, the third pressure range may be a high level pressure range.

In the present disclosure, at least one of the skin care tips 100, 500, or 900 may be replaceably mounted on the hand piece. At this time, the hand piece may include a control module for controlling at least one of the skin care tips 100, 500, or 900.

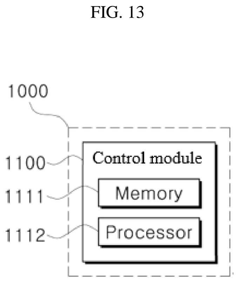

FIG. 13 illustrates a configuration of a control module of a hand piece equipped with a skin care tip according to the present disclosure.

Referring to FIG. 13, a control module 1100 of a hand piece 1000 may be implemented with a memory 1111 that stores data on an algorithm for controlling the operation of components within the device or a program that reproduces the algorithm, and at least one processor 1112 that performs the operation described above using the data stored in the memory 1111. Here, the memory 1111 and the processor 1112 may be implemented as separate chips, respectively. In addition, the memory 1111 and the processor 1112 may be implemented as a single chip.

The memory 1111 may store data supporting various functions of the device, a program for the operation of the control module, may store input/output data, and may store a plurality of application programs application programs or applications driven by the device, data for the operation of the device, and commands. At least some of these applications may be downloaded from an external server via wireless communication.

The memory 1111 may include at least one type of storage medium among a flash memory type, a hard disk type, an SSD type (Solid State Disk type), an SDD type (Silicon Disk Drive type), a multimedia card micro type, a card type memory (e.g., an SD or XD memory, etc.), random access memory (RAM), static random access memory (SRAM), read only memory (ROM), electrically erasable programmable read-only memory (EEPROM), programmable read-only memory (PROM), a magnetic memory, a magnetic disk, and an optical disk. In addition, the memory 1111 may be a database that is separate from the device but connected by wire or wirelessly.

The processor 1112 may adjust the opening speed of at least one of the first to third valves 170, 570, or 970 when the pressure of the cooling gas is in a preset first pressure range. In this case, the processor 1112 may adjust the opening speed of at least one of the first to third valves 170, 570, or 970 corresponding to the first pressure range to perform a rapid opening or an average opening or a delayed opening operation at a preset micro-opening level in conjunction with the first pressure range.

The processor 1112 may adjust the opening speed of at least one of the first to third valves 170, 570, or 970 when the pressure of the cooling gas is in a second pressure range higher than the preset first pressure range. In this case, the processor 1112 may adjust the opening speed of at least one of the first to third valves 170, 570, or 970 set corresponding to the second pressure range to perform a fast opening or average opening or delayed opening operation at a preset half-opening level in conjunction with the second pressure range.

The processor 1112 may also adjust the opening speed of at least one of the first to third valves 170, 570, or 970 when the pressure of the cooling gas is in a third pressure range higher than the preset second pressure range. In this case, the processor 1112 may adjust the opening speed of at least one of the first to third valves 170, 570, or 970 set corresponding to the third pressure range to perform a fast opening or average opening or delayed opening operation at a preset full-opening level in conjunction with the third pressure range.

At least one of first to third injection control modules 180, 580, or 980 of the skin care tip 100, 500, and 900 according to the present disclosure is mounted on at least one of the first to third injection parts 130, 530, or 930, and may control the injection amount of the cooling gas according to the pressure of the cooling gas. In this case, at least one of the first to third injection control modules 180, 580, or 980 may be provided as a fourth valve inside at least one of the first to third injection parts 130, 530, or 930. For example, the fourth valve may be provided as a check valve, but is not limited thereto, and may be provided as a solenoid valve. As another example, the fourth electronic valve may be provided with a check valve and a solenoid valve together.

Here, the processor 1112 may control the injection amount of the cooling gas by controlling at least one of the first to third injection control modules 180, 580, or 980 according to the pressure of the cooling gas. For example, when the pressure of the cooling gas is in a preset first pressure range, the processor 1112 may adjust the opening state of at least one of the first to third injection control modules 180, 580, or 980 corresponding to the first pressure range so that the cooling gas is injected at a preset first injection amount level in conjunction with the first pressure range. For another example, the processor 1112 may adjust the opening state of at least one of the first to third injection control modules 180, 580, or 980 set corresponding to the second pressure range so that the cooling gas is injected at the second injection level lower than the first injection level set in conjunction with the second pressure range when the pressure of the cooling gas is in the second pressure range higher than the first pressure range. For another example, the processor 1112 may adjust the opening state of at least one of the first to third injection control modules 180, 580, or 980 set corresponding to the third pressure range so that the cooling gas is injected at the third injection level lower than the second injection level set in conjunction with the third pressure range when the pressure of the cooling gas is in the third pressure range higher than the second pressure range. Here, the first injection level may be higher than the second injection level, and the second injection level may be higher than the third injection level. At this time, the open state may be at least one of the open degree and the open speed.

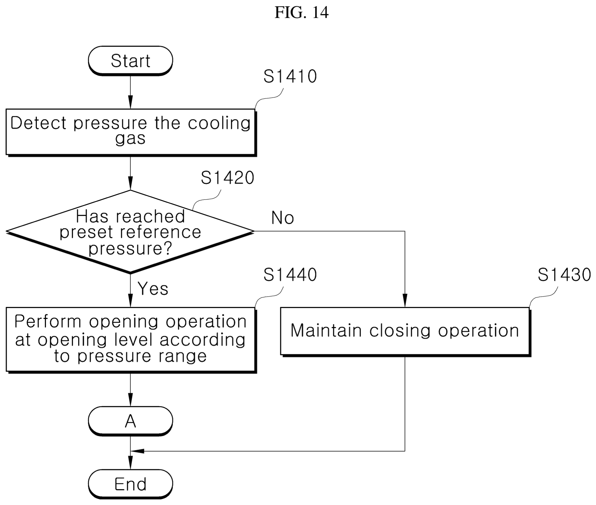

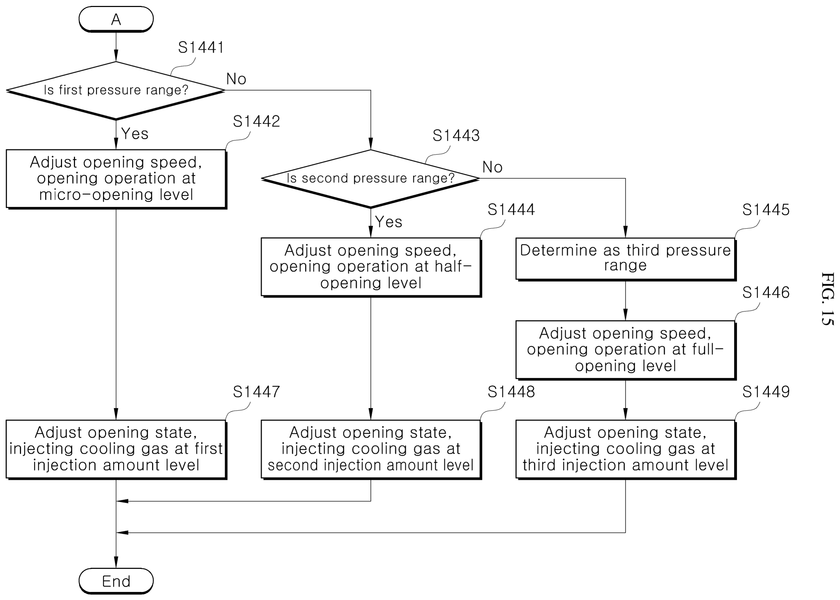

FIGS. 14 and 15 are flowcharts illustrating an example of an operation method of a valve included in a skin care tip according to the present disclosure. Referring to FIG. 14, an operation method of at least one of the first to third valves 170, 570, or 970 may include a detection step S1410, a determination step S1420, a maintenance step S1430, and an opening operation step S1440.

The detection step may detect the pressure of the cooling gas injected through the refrigerant delivery path A11 to A18, A21 to A29, and A31 to A42 of the skin care tip 100, 500, and 900 through at least one of the first to third valves 170, 570, or 970 (step S1410).

The determination step may determine whether the pressure of the cooling gas has reached the preset reference pressure through at least one of the first to third valves 170, 570, or 970 (step S1420).

The maintenance step may maintain the closing operation in the case that the pressure of the cooling gas does not reach the preset reference pressure through at least one of the first to third valves 170, 570, or 970 (step S1430).

The opening operation step may perform the opening operation at an opening level according to the preset pressure range in the case that the pressure of the cooling gas reaches the reference pressure through at least one of the first to third valves 170, 570, or 970 (step S1440).

For example, at least one of the first to third valves 170, 570, or 970 may open at a preset micro-opening level in conjunction with the first pressure range when the pressure of the cooling gas is in a preset first pressure range. In this case, the first pressure range may be a low level pressure range.

For another example, at least one of the first to third valves 170, 570, or 970 may open at a preset half-opening level in conjunction with the second pressure range when the pressure of the cooling gas is in the second pressure range higher than the preset first pressure range. In this case, the second pressure range may be a middle level pressure range.

For another example, at least one of the first to third valves 170, 570, or 970 may open to a preset full-opening level in conjunction with the third pressure range when the pressure of the cooling gas is in the third pressure range higher than the preset second pressure range. In this case, the third pressure range may be a high level pressure range.

Referring to FIG. 15, the operating method of the valve may include a first determination step S1441, a first opening operation step S1442, a second determination step S1443, a second opening operation step S1444, a third determination step S1445, and a third opening operation step S1446.

The first determination step may determine whether the pressure of the cooling gas is within the preset first pressure range through the processor 1112 of the hand piece 1000 (step S1441).

The first opening operation step may adjust the opening speed of at least one of the first to third valves 170, 570, or 970 when the pressure of the cooling gas is within the preset first pressure range through the processor 1112. At this time, the processor 1112 may adjust the opening speed of at least one of the first to third valves 170, 570, or 970 corresponding to the first pressure range to perform a quick opening or average opening or delayed opening operation at a preset micro-opening level in conjunction with the first pressure range (step S1442).

The second determination step may determine whether the pressure of the cooling gas is in the second pressure range higher than the preset first pressure range through the processor 1112 (step S1443).

The second opening operation step may adjust the opening speed of at least one of the first to third valves 170, 570, or 970 when the pressure of the cooling gas is within the preset second pressure range through the processor 1112. In this case, the processor 1112 may adjust the opening speed of at least one of the first to third valves 170, 570, or 970 corresponding to the second pressure range to perform a rapid opening or an average opening or a delayed opening operation at a preset half-opening level in conjunction with the second pressure range (step S1444).

The third determination step may determine, through the processor 1112, that the pressure of the cooling gas is within the preset second pressure range in the case of not within the third pressure range (step S1445).

The third opening operation step may adjust the opening speed of at least one of the first to third valves 170, 570, or 970 when it is determined to be the third pressure range through the processor 1112. In this case, the processor 1112 may adjust the opening speed of at least one of the first to third valves 170, 570, or 970 corresponding to the third pressure range to perform a fast opening or an average opening or delayed opening operation at a preset full-opening level in conjunction with the third pressure range (step S1446).

In addition, the operating method of the valve according to the present disclosure may include a first injection step S1447, a second injection step S1448, and a third injection step S1449.

In the first injection step, when the pressure of the cooling gas is in the preset first pressure range (step S1441), the opening state of at least one of the first to third injection control modules 180, 580, or 980 set corresponding to the first pressure range may be adjusted so that the cooling gas is injected at a preset first injection amount level linked to the first pressure range (step S1447).

In the second injection step, when the pressure of the cooling gas is in the second pressure range higher than the preset first pressure range (step S1443), the opening state of at least one of the first to third injection control modules 180, 580, or 980 set corresponding to the second pressure range may be adjusted so that the cooling gas is injected at a second injection amount level lower than the preset first injection amount level linked to the second pressure range (step S1448).

The third injection step may adjust the opening state of at least one of the first to third injection control modules 180, 580, or 980 corresponding to the third pressure range so that the cooling gas is injected at a third injection amount level lower than the second injection amount level set in conjunction with the third pressure range when the pressure of the cooling gas is in the third pressure range higher than the second pressure range (step S1445), through the processor 1112 (step S1449). Here, the first injection amount level may be higher than the second injection amount level, and the second injection amount level may be higher than the third injection amount level. In this case, the opening state may be at least one of the opening degree and the opening speed.

The present disclosure may control at least one of the first to third injection control modules 180, 580, or 980 to prevent at least one of the first to third injection parts 130, 530, or 930 from freezing by injecting cooling gas several times by a preset number of times through the processor 1112.

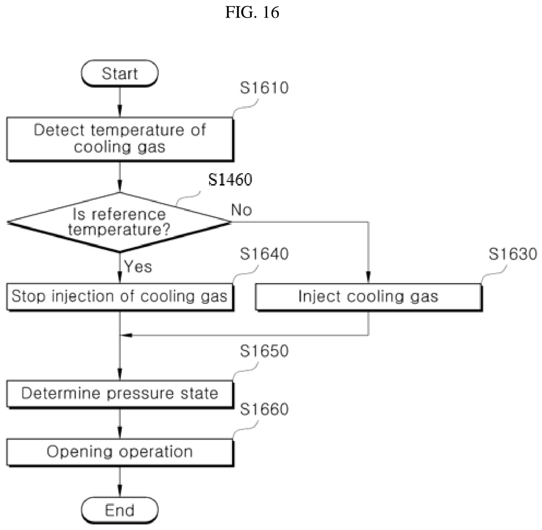

FIG. 16 is a flowchart illustrating another example of an operation method of a valve included in a skin care tip according to the present disclosure.

Referring to FIG. 16, the operation method of at least one of the first to third valves 170, 570, or 970 may include a detection step S1610, a first determination step S1620, an injection step S1630, an injection stop step S1640, a second determination step S1650, and an opening operation step S1660.

The detection step may detect the temperature of the cooling gas through a temperature sensor provided in at least one of the first to third electrode members 120, 520, or 920 (step S1610). In this case, the location of the temperature sensor is not limited thereto, and may be provided on an inner side of at least one of the first to third housings 110, 510, or 910.

The first determination step may determine whether the temperature of the cooling gas is a preset reference temperature through the processor 1112 (step S1620).

The injection step may control the injection nozzle that injects the cooling gas so that the temperature of the cooling gas is lowered to the preset reference temperature through the processor 1112 in the case that the temperature of the cooling gas is not the preset reference temperature (NO in step S1620) (step S1630). In this case, the processor 1112 may control the injection nozzle to inject a preset cooling gas according to the temperature of the cooling gas. That is, the processor 1112 may control the injection nozzle to inject a large amount of cooling gas when the temperature of the cooling gas is high.

The injection stop step may control the injection nozzle to stop the injection of the cooling gas through the processor 1112 in the case that the temperature of the cooling gas is a preset reference temperature (example of step S1620) (step S1640).

The second determination step may determine the pressure state that occurs when the cooling gas remaining in the receiving space of at least one of the first to third housings 110, 510, or 910 pressurizes at least one of the first to third valves 170, 570, or 970 through at least one of the first to third valves 170, 570, or 970 (step S1650). In this case, the pressure state may vary depending on the injection amount of the cooling gas. For example, at least one of the first to third valves 170, 570, or 970 may be determined as the first pressure state in the case that the injection amount of the cooling gas is within the preset first injection amount range, may be determined as the second pressure state in the case that the second injection amount range is greater than the preset first injection amount range, and may be determined as the third pressure state in the case that the third injection amount range is greater than the preset second injection amount range. In this case, the first pressure state may be a low level pressure state, the second pressure state may be a middle level pressure state, and the third pressure state may be a high level pressure state.

The opening operation step may perform the opening operation when the pressure state reaches the preset target pressure through at least one of the first to third valves 170, 570, or 970 (step S1660). For example, at least one of the first to third valves 170, 570, or 970 may perform an opening operation with a first condition of at least one of an opening level and an opening speed corresponding to a low-level pressure state when the first pressure state reaches a first target pressure that is set to a preset first target pressure. For another example, at least one of the first to third valves 170, 570, or 970 may perform an opening operation with a second condition of at least one of an opening level and an opening speed corresponding to a middle level pressure state when the second pressure state reaches a second target pressure that is higher than the first target pressure. For yet another example, at least one of the first to third valves 170, 570, or 970 may perform an opening operation with a third condition of at least one of an opening level and an opening speed corresponding to a high level pressure state when the third pressure state reaches a third target pressure that is higher than the second target pressure. In this case, the third condition may be set to at least one of a wider opening level and a faster speed than the second condition, and the second condition may be set to at least one of a wider opening level and a faster speed than the first condition.

Therefore, the operating method of the tip and valve for skin care according to the present disclosure, and the hand piece including the same, may prevent pain and burns due to heat generation in the high-frequency electrode in advance, prevent an increase in the cost of using a refrigerant in advance, and improve cooling efficiency by maintaining the internal temperature low.

At least one component may be added or deleted corresponding to the performance of the components illustrated in FIGS. 1 to 13. In addition, it will be readily understood by those skilled in the art that the mutual positions of the components may be changed corresponding to the performance or structure of the system.

Although FIG. 14 and FIG. 15 describe the execution of multiple steps sequentially, this is only an exemplary description of the technical idea of the present embodiment, and a person having ordinary knowledge in the technical field to which the present embodiment belongs may modify and change the order described in FIG. 14 and FIG. 15 without departing from the essential features of the present embodiment, or may modify and change one or more of the multiple steps in parallel, and thus FIG. 14 and FIG. 15 are not limited to a chronological order.

As described above, the disclosed embodiments have been described with reference to the attached drawings. A person having ordinary knowledge in the technical field to which the present disclosure belongs will understand that the present disclosure may be implemented in a form different from the disclosed embodiments without changing the technical idea or essential features of the present disclosure. The disclosed embodiments are exemplary and should not be construed as limiting.

## Claims

1. A skin care tip comprising:
a housing;
an injection part provided in the housing and including a refrigerant delivery path formed to inject a cooling gas;
a discharge part coupled to the injection part and including a discharge path formed to discharge the cooling gas to outside; and
a valve coupled to the injection part and the discharge part and configured to block external air flowing in through the refrigerant delivery path and discharge the cooling gas to the outside through the discharge path while retaining the cooling gas, and configured to open and close according to a pressure of the cooling gas.

2. The skin care tip according to claim 1, further comprising an injection control module mounted on the injection part and controlling an amount of the cooling gas injected according to the pressure of the cooling gas.

3. The skin care tip according to claim 1, wherein the valve maintains a closing operation until the pressure of the cooling gas reaches a preset reference pressure, and opens when the reference pressure is reached.

4. The skin care tip according to claim 3, wherein the valve opens at an opening level according to a preset pressure range when the pressure of the cooling gas reaches the reference pressure.

5. The skin care tip according to claim 4, wherein,
based on the pressure of the cooling gas being in a preset first pressure range, the valve opens at a preset micro-opening level in conjunction with the first pressure range.

6. The skin care tip according to claim 4, wherein,
based on the pressure of the cooling gas being in a preset second pressure range, the valve opens at a preset half-opening level in conjunction with the second pressure range.

7. The skin care tip according to claim 4, wherein,
based on the pressure of the cooling gas being in a preset third pressure range, the valve opens at a preset full-opening level in conjunction with the third pressure range.

8. The skin care tip according to claim 1, wherein the valve is at least one of a check valve or a solenoid valve.

9. A hand piece equipped with the skin care tip according to any one of claims 1 to 8.

10. A method of operating a valve included in a skin care tip, comprising:
detecting a pressure of a cooling gas injected through a refrigerant delivery path of the skin care tip;
maintaining a closing operation when a pressure of the cooling gas does not reach a preset reference pressure; and
opening at an opening level according to a preset pressure range when the pressure of the cooling gas reaches the preset reference pressure.
